# EUROPÄISCHE PATENTSCHRIFT

(11) **EP 1 260 200 B1**
(45) Veröffentlichungstag und Bekanntmachung des Hinweises auf die Patenterteilung: **28.03.2007**
(21) Anmeldenummer: 02011045.8
(22) Anmeldetag: 17.05.2002
(51) Int. Cl.: A61F 2/36, A61F 2/30

(54) **Zementfreie Hüftgelenksendoprothese als Oberflächenersatz des proximalen Femurs**
Cementless hip joint endoprosthesis for replacing the surface of the proximal femur
Endoprothèse non cimentée de l'articulation de la hanche pour remplacer la surface de la partie proximale du fémur

(30) Priorität: 18.05.2001 DE 10124705; 23.06.2001 DE 10130366
(43) Veröffentlichungstag der Anmeldung: 27.11.2002
(73) Patentinhaber: Copf, Peter, Dr. med, 72074 Tübingen (DE); Hein, Werner, Dr. med., 04289 Leipzig (DE); Kranz, Curt, Dr., 14163 Berlin (DE); Copf, Franz, Prof., 70184 Stuttgart (DE)
(72) Erfinder: Copf, Peter, Dr. med., 72074 Tübingen (DE); Hein, Werner, Prof. Dr. med., 04289 Leipzig (DE)
(74) Vertreter: Dreiss, Fuhlendorf, Steimle & Becker

(56) Entgegenhaltungen:
- EP-A- 0 668 064
- EP-A- 0 765 644
- EP-A- 0 768 066
- WO-A-00/72785
- WO-A-96/07374
- WO-A-96/22746
- WO-A-98/26725
- DE-A- 3 006 178
- DE-A- 19 635 307
- DE-A- 19 904 126
- DE-C- 923 383
- DE-U- 29 921 577
- FR-A- 2 391 712
- FR-E- 59 919
- GB-A- 764 600
- GB-A- 2 139 097
- US-A- 4 808 186
- US-A- 5 702 448
- US-A- 5 709 020
- US-A- 6 096 084
- US-B1- 6 228 123

## Beschreibung

Die Erfindung betrifft eine Hüftgelenksendoprothese als Oberflächenersatz des proximalen Femurs mit einer Kalotte, wobei die Kalotte eine mit einer Gelenkpfanne zusammenwirkende Außenfläche und eine Innenfläche aufweist und mit einem Schaft, wobei der Schaft mit der Kalotte verbindbar ist.

Diese Hüftgelenksendoprothesen werden nach dem Stand der Technik in den Oberschenkelhals einzementiert. Durch die Verwendung von Zement zur Befestigung der Hüftgelenksendoprothese kann eine Vielzahl von Problemen für den Patienten entstehen. Bspw. kann durch die unterschiedlichen Temperatur-Ausdehnungskoeffizienten von Knochen, Zement und Hüftgelenksendoprothese sowie der Alterung des Zements eine Lockerung der Hüftgelenksendoprothese im Oberschenkelhals eintreten. Außerdem kann die Festigkeit der Verbindung von Hüftgelenksendoprothese und Oberschenkelhals durch eine Volumenänderung des Zements beim Aushärten von Anfang an verringert sein. Schließlich kann der Zement auch zu Abstoßungsreaktionen führen. All diese möglicherweise auftretenden Probleme, die durch die Verwendung von Zement ausgelöst werden können, sind aus der einschlägigen Fachliteratur bekannt.

Dokument DE 30 06 178 offenbart eine Hüftgelenksendoprothese nach dem Oberbegriff von Anspruch 1.

Der Erfindung liegt die Aufgabe zu Grunde, eine Hüftgelenksendoprothese als Oberflächenersatz des proximalen Femurs bereitzustellen, die ohne Verwendung von Zement eine sichere, belastbare, dauerhafte und verträgliche Befestigung der Hüftgelenksendoprothese im Oberschenkelhals ermöglicht.

Diese Aufgabe wird bei einer Hüftgelenksendoprothese mit einer Kalotte, wobei die Kalotte eine mit einer Gelenkpfanne zusammenwirkende Außenfläche und eine Innenfläche aufweist und mit einem Schaft, wobei der Schaft mit der Kalotte verbindbar ist, dadurch gelöst, dass der Schaft eine gitterartige Struktur aufweist.

Bei der Implantation der erfindungsgemäßen Hüftgelenksendoprothese wird die gitterartige Struktur im Bereich der Spongiosa des Oberschenkelhalse positioniert. Dadurch besteht die Möglichkeit, dass die zuvor mindestens teilweise entfernte Spongiosa im Bereich des Oberschenkelhalses nach der Implantation in die gitterartige Struktur einwächst und somit eine hochbelastbare und in ausreichendem Maße elastische Verbindung zwischen Schaft und Oberschenkelhals herstellt. Eine gewisse Elastizität der Verbindung zwischen Hüftgelenksendoprothese und Oberschenkelhals ist erforderlich, um die Spongiosa zum Wachstum und zur Regeneration über die gesamte Lebensdauer der Hüftgelenksendoprothese anzuregen. Dadurch ist über die gesamte Lebensdauer der Hüftgelenksendoprothese ein sicherer und belastbarer Sitz der Hüftgelenksendoprothese im Oberschenkelhals gewährleistet. Zusätzlich zu den genannten Vorteilen der Erfindung, hat die erfindungsgemäße Hüftgelenksendoprothese alle aus dem Stand der Technik bekannten Vorteile eines Oberflächenersatzes der Gelenkkugel. Einer dieser Vorteile besteht darin, dass der Oberschenkelhals nicht entfernt werden muss, so dass nach dem Ablauf der Lebensdauer der erfindungsgemäßen Hüftgelenksendoprothese eine zweite Hüftgelenksendoprothese einsetzbar ist, bei der der Oberschenkelhals entfernt wird. Außerdem ist der Ersatz der Oberfläche des Kopfes des Oberschenkels wesentlich weniger traumatisch als der Einsatz einer Hüftgelenksendoprothese, bei der der Oberschenkelhals entfernt werden muss.

Erfindungsgemäß kann vorgesehen sein, dass Kalotte und Schaft in implantiertem Zustand miteinander kraft- oder formschlüssig verbunden sind, oder dass in implantiertem Zustand sich die Kalotte auf dem Schaft abstützt.

Bei der erfindungsgemäßen Hüftgelenksendoprothese erfolgt die Verankerung nahezu ausschließlich über die Spongiosa des Oberschenkelhalses. Eine Krafteinleitung in die Kortikalis des Oberschenkelhalses ist nut in sehr geringem Umfang vorgesehen, da diese Krafteinleitung, wegen der größeren Festigkeit der Kortikalis, dazu führen würde, dass keine oder nur noch sehr geringe Kräfte in die Spongiosa eingeleitet werden und in Folge dessen eine Stimulation des Wachstums der Spongiosa nicht oder nicht in ausreichendem Maße erfolgen würde. Dies hätte langfristig eine Lockerung der Hüftgelenksendoprothese im Oberschenkelhals zur Folge.

Besonders vorteilhafterweise ist die Steifigkeit des Schaftes, insbesondere in Längsrichtung des Schaftes, größer als die den Schaft umgebende Spongiosa und/oder ist die Steifigkeit des Schaftes, insbesondere in Längsrichtung des Schaftes, kleiner als die Kortikalis des Oberschenkelhalses. Bei dieser Auslegung des Schaftes wird eine ausreichend große Kraft von der Kalotte über den Schaft in die Spongiosa eingeleitet, um die Resektionsflächen des Femurkopfes zu entlasten und eine dem natürlichen Knorpelgewebe so weit wie mögliche entsprechende Dämpfung zu erzielen. Außerdem wird die Spongiosa durch die Krafteinleitung zum Wachstum angeregt, ohne die Spongiosa zu überfordern. Aus der Literatur (Siehe Röhrle, H.; Scholten, W.; Grünert, A.: "Der Kraftfluß bei Hüftendoprothesen" in Arch. orthop. Unfall-Cir. 89, 49-70, 1977) ist es bekannt, dass der Elastizitätsmodul der Spongiosa in dem Bereich in den der Schaft verankert wird etwa 1,21 x 10⁵ N/cm² und für die Kortikalis des Oberschenkelhalses etwa 1,45 x 10⁶ N/cm² beträgt.

Weitere Varianten der Erfindung sehen vor, dass sich die gitterartige Struktur des Schaftes aus Öffnungen oder Stäben und Streben zusammensetzt, und/oder dass die gitterartige Struktur Verdickungen und/oder Vertiefungen oder eine maschenartige Struktur aufweist, so dass ein möglichst gutes Einwachsen der Spongiosa in die gitterartige Struktur im Sinne einer festen und gleichzeitig elastischen Verbindung zwischen Hüftgelenksendoprothese und Oberschenkelhals gewährleistet ist. Neben einem verbesserten Formschluß vergrößern die Verdickungen und Vertiefungen die Oberfläche des Schaftes, was sich vorteilhaft auf die Festigkeit der Verankerung der Hüftgelenksendoprothese auswirkt.

Es hat sich weiterhin als vorteilhaft erwiesen, wenn die Längsachse des Schaftes in Richtung der höchsten Beanspruchung des Oberschenkelhalses verläuft, so dass der Schaft diese Beanspruchung aufnehmen kann und über seine gesamte Länge, bzw. Oberfläche, in die Spongiosa des Oberschenkelhalses einleiten kann.

Das Einwachsen der Spongiosa in die Endoprothese erfolgt in drei Phasen: In der ersten Phase dürfen nur kleine Kräfte von der Endoprothese auf die Spongiosa übertragen werden (physiologische Krafteinleitung). Deshalb ist eine relative Ruhigstellung der einander berührenden Oberflächen von Endoprothese und Spongiosa erforderlich. Außerdem müssen die Resektionsflächen sehr genau mit der Endoprothese zusammenpassen. In dieser ersten Phase ist der Schaft zur Sicherstellung einer guten Kräfteverteilung/Krafteinleitung wichtig; er entlastet die konische Auflagefläche der Kalotte.

Wenn sich in der zweiten Phase die konische Auflagefläche der Kalotte mit der Spongiosa verbunden hat (Knochenaufbau und damit Versteifung der Umgebung der Kalotte), lässt die Kraftaufnahme des Schaftes nach (verlagerung des Kraftflusses auf die Kalotte).

Wenn die Spongiosa auf Dauer (dritte Phase) nicht in der Lage ist, die auftretenden Kräfte aufzunehmen, wird der Schaft dauerhaft einen Teil dieser Kräfte in die Spongiosa einleiten. Um zu verhindern, dass der Schaft die gesamten auf die Kalotte einwirkenden Kräfte übernimmt, ist es vorteilhaft, wenn der Schaft in folgender Weise ausgeführt wird: a.) Das erste Drittel des Schaftes soll biegesteif mit der Kalotte verbunden sein, in Längsrichtung jedoch möglichst eine in den o g. Grenzen liegende Steifigkeit aufweisen. b.) das zweite Drittel des Schaftes soll biegeweich sein und in Längsrichtung eine in den o g. Grenzen liegende Steifigkeit aufweisen. c.) das dritte Drittel des Schaftes soll biegeweich sein und in Längsrichtung eine überhalb der o g. Grenzen liegende Steifigkeit aufweisen.

Zur Verbesserung der Verbindung von Hüftgelenksendoprothese und Oberschenkelhals kann alternativ vorgesehen sein, dass die Oberfläche des Schaftes porös, periplasmal oder hydroxyapatit ist. Wenn die Oberfläche porös oder periplasmal ist, kann die Spongiosa in die Oberfläche einwachsen, was zu einer entsprechend guten Verzahnung und Verbindung zwischen Spongiosa und Hüftgelenksendoprothese führt. Wenn die Oberfläche der gitterartigen Struktur, bzw. des Schaftes, hydroxyapatit ist, kann die Spongiosa an der Oberfläche anwachsen, was auch eine feste und dauerhaft belastbare Verbindung von Hüftgelenksendoprothese und Oberschenkelhals ermöglicht.

Zur weiteren Verbesserung der Krafteinleitung in den Oberschenkelhals ist vorgesehen, dass die Innenfläche der Kalotte der Oberfläche eines doppelten Kegelstumpfes entspricht, da bei dieser Ausgestaltung der erfindungsgemäßen Hüftgelenksendoprothese durch die Drehbewegung eines entsprechend geformten Werkzeugs die Resektionsflächen einfach und in der erforderlichen Genauigkeit herstellbar sind. Durch eine geeignete Wahl der Kegelwinkel kann die gewünschte Klemmwirkung zwischen Kalotte und den Resektionsflächen des Oberschenkelhalsknochens erreicht werden. Diese Klemmwirkung ist insbesondere unmittelbar nach der Operation von besonderer Bedeutung für die Verankerung der Hüftgelenksendoprothese im Oberschenkelhals. Nach dem Einwachsen der Spongiosa in die gitterartige Struktur liegt die Bedeutung der doppeltkegelstumpfförmigen Kontakflächen zwischen Kalotte und Knochen darin, eine möglichst flächige Krafteinleitung in den Oberschenkelhals zu gewährleisten. Dadurch werden die auf die gitterartige Struktur und die mit dieser Struktur verwachsene Spongiosa wirkenden Belastungen auf das notwendige Maß reduziert.

Um eine möglichst gute Krafteinleitung von der Hüftgelenksendoprothese in den Oberschenkelhals zu gewährleisten ist vorgesehen, dass der Kopf des Oberschenkels soweit abgetragen wird, dass die Kortikalis vollständig entfernt wird und eine Verankerung der Hüftgelenksendoprothese im Bereich des Kopfes des Oberschenkels ausschließlich über die Spongiosa und nicht über die Kortikalis erfolgt. Allerdings ist dabei zu darauf zu achten, dass die Tragfähigkeit des Kopfes des Oberschenkelhalsknochens nicht zu sehr verringert wird.

Zur Vereinfachung der Herstellung von Kalotte und Schaft sowie zur Vereinfachung der Implantation hat es sich als vorteilhaft herausgestellt, dass die Kalotte und der Schaft durch eine Klemmverbindung miteinander verbunden sind. Dies kann beispielsweise durch eine konische Ausnehmung im Schaft und einen entsprechenden Zapfen an der Kalotte erreicht werden. Der Querschnitt der konischen Ausnehmung und des Zapfens kann beispielsweise rund rund, polygonal, rechteckig oder quadratisch sein.

Schließlich kann die Außenfläche der Kalotte mit einer verschleißmindernden und/oder reibungsverringernden Beschichtung versehen sein, um die Lebensdauer der erfindungsgemäßen Hüftgelenksendoprothese zu erhöhen.

Es hat sich als vorteilhaft herausgestellt die Hüftgelenksendoprothese mindestens teilweise aus Titan, insbesondere TiAl6V4, Stahl oder einer CoCr-Legierung durch Gießen und/oder Schmieden herzustellen. Dabei kann die Kalotte u. a. wegen der guten tribologischen Eigenschaften aus Stahl oder einer CoCr-Legierung hergstellt werden, während der Schaft aus TiAl6V4 bestehen kann. Wahlweise kann die Hüftgelenksendoprothese mindestens teilweise aus Sintermetall oder poröser Keramik oder biologisch abbaubarem Material bestehen.

Weitere Vorteile und vorteilhafte Ausgestaltungen der Erfindung sind den nachfolgenden Zeichnungen, deren Beschreibung und den Patentansprüchen entnehmbar.

### Zeichnung

Es zeigen
- Figur 1: ein proximales Femur im Längsschnitt,
- Figur 2: ein proximales Femur mit einem für die Implantation einer erfindungsgemäßen Hüftgelenksendoprothese vorbereiteten Kopf,
- Figur 3a, b: ein erstes Ausführungsbeispiel einer erfindungsgemäßen Hüftgelenksendoprothese,
- Figur 4: ein zweites Ausführungsbeispiel eines Schafts einer erfindungsgemäßen Endoprothese,
- Figur 5: ein zweites Ausführungsbeispiel einer erfindungsgemäßen Hüftgelenksendoprothese im Schnitt und
- Figur 6a, b: ein Modell zur Veranschaulichung der erfindungsgemäßen Krafteinleitung in den Oberschenkelhals.

### Beschreibung der Ausführungsbeispiele

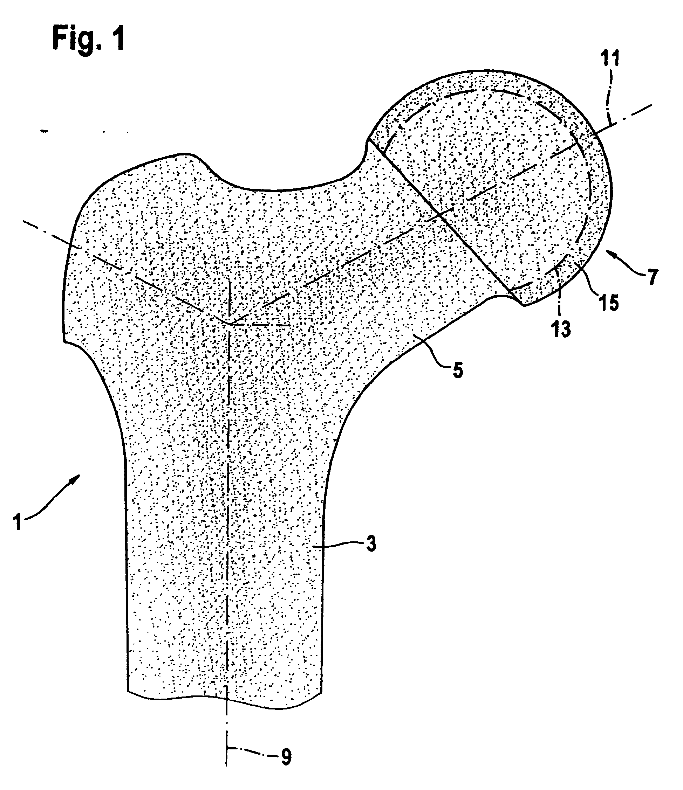

Figur 1 zeigt ein proximales Femur 1 im Längsschnitt. An einem Oberschenkelknochen 3 des Femurs 1 schließt ein Oberschenkelhals 5 mit einem Kopf 7 an. Eine Längsachse 9 des Oberschenkelknochens 3 sowie eine Längsachse 11 des Oberschenkelhalses 5 schließen einen Winkel von etwa 120° ein. Durch eine strichpunktierte Linie 13 ist die Grenze zwischen Kortikalis 15 und der nicht dargestellten Spongiosa im Bereich des Kopfes 7 dargestellt.

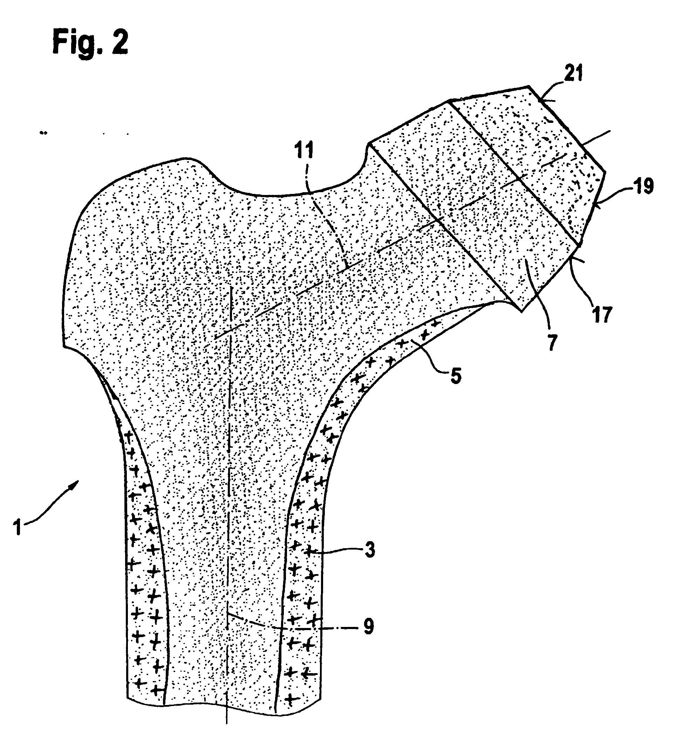

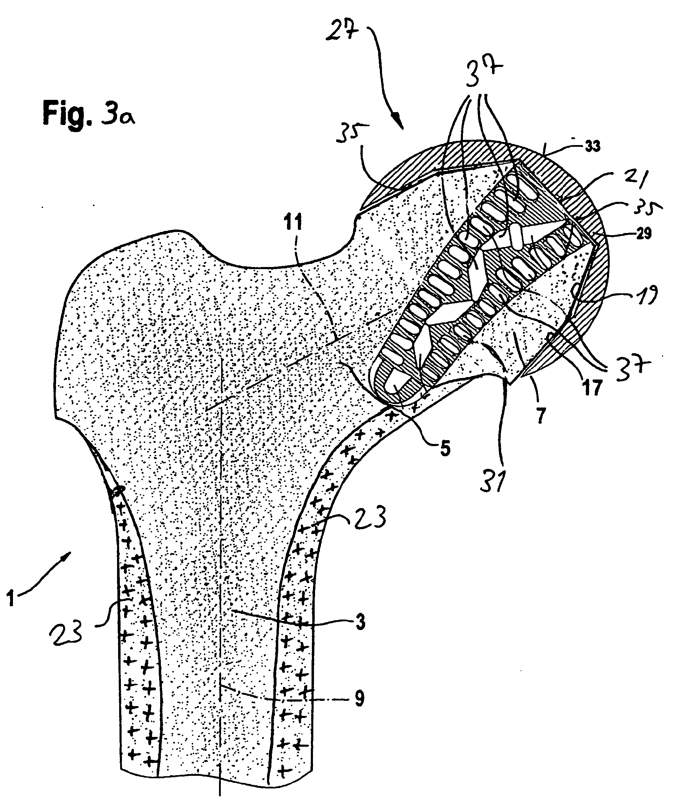

Wenn das aus Kopf 7 und einer nicht dargestellten Gelenkpfanne bestehende Hüftgelenk ausgewechselt werden muss, wird der Kopf des Femurs entsprechend Figur 2 vorbereitet. Aus Figur 2 ist ersichtlich, dass der Kopf 7 so abgetragen wurde, dass er die Gestalt doppelten Kegelstumpfes annimmt. Mit dem Bezugszeichen 17 ist ein erster Kegelstumpf gekennzeichnet, während das Bezugszeichen 19 einen daran anschließenden zweiten Kegelstumpf kennzeichnet. An den zweiten Kegelstumpf 18 schließt sich eine Stirnfläche 21 an. Der rotationssymmetrische doppelte Kegelstumpf 17, 19 lässt sich relativ einfach und mit guter Genauigkeit auch unter Operationsbedingungen herstellen. Dabei wird so viel Material abgetragen, dass die Kortikalis 15 (siehe Fig. 1) des Kopfes 7 vollständig entfernt wird. In anderen Worten: Der entsprechend Figur 2 vorbereitete Kopf 7 besteht ausschließlich aus Spongiosa. Die Zahl der Kegelstümpfe 17, 19 sowie deren KegelWinkel ist erfindungsgemäß nicht auf dieses Ausführungsbeispiel beschränkt.

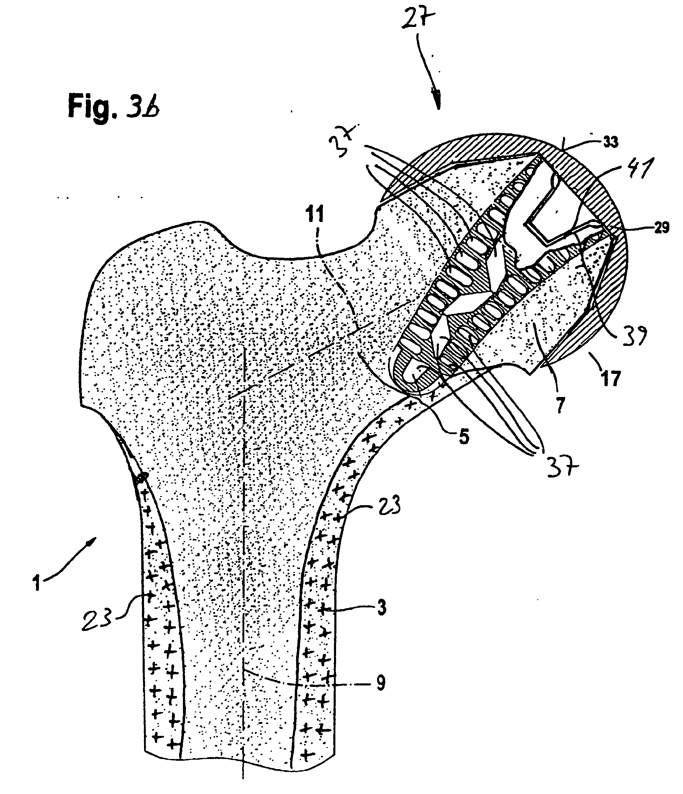

In Figur 3a ist ein erstes Ausführungsbeispiel einer erfindungsgemäßen Hüftgelenksendoprothese 27 auf den gemäß Fig. 2 vorbereiteten Kopf 7 aufgesetzt dargestellt. Die Hüftgelenksendoprothese 27 besteht aus einer Kalotte 29 und einem Schaft 31. Die Kalotte 29 weist eine kugelförmige Außenfläche 33 und eine doppelt kegelstumpfförmig ausgestaltete Innenfläche 35 auf. Durch den Vergleich der Figuren 2 und 3a wird deutlich, dass die Innenfläche 35 der Kalotte 29 die gleiche Form wie der vorbereitete Kopf 7 aufweist. Dadurch wird eine kraft- und formschlüssige Verbindung zwischen Kalotte 29 und dem Kopf 7 hergestellt, wenn die Hüftgelenksendoprothese 27 in den Oberschenkelhals 5 implantiert wird.

Der Schaft 31 weist eine gitterartige Struktur mit verschieden angeordneten und verschieden ausgerichteten Öffnungen 37 auf. Aus Gründen der Übersichtlichkeit sind in Figur 3 nicht alle Öffnungen mit Bezugszeichen versehen. Hinsichtlich Form, Ausrichtung und Größe der Öffnungen 37 ist darauf zu achten, dass die Spongiosa möglichst leicht in die Öffnungen einwachsen kann und eine große Oberfläche des Schaftes 31 zur Verfügung steht. Prinzipiell gilt: Je größer die Oberfläche des Schaftes 31 ist, desto besser ist die Verbindung zwischen Spongiosa und Schaft 31, wenn die Spongiosa eingewachsen ist.

Bei der Auslegung des Schaftes 31 ist ebenso darauf zu achten, dass der Schaft eine gewisse Elastizität aufweist, um erstens örtliche, auf den Resektionsbereich wirkende Belastungsspitzen zu vermeiden, indem ein Teil der auf die Kalotte wirkenden Kräfte vom Schaft 31 aufgenommen werden und dadurch die auf die Resektionsflächen des Femurkopfes 7 wirkenden Kräfte verringert werden. Der Schaft 31 hat somit die wichtige Funktion eines Stoßdämpfers, wobei die Dämpfung durch die Reibung zwischen Schaft und Spongiosa sowie durch die Umströmung der gitterartigen Struktur des Schaftes durch die visköse Flüssigkeit, welche sich in den Hohlräumen der Spongiosa befindet und in die implantierte Schaftstruktur einfließt, erfolgt. Im zeitlichen Verlauf des Einwachsens der Kalotte wird der Schaft nach und nach überflüssig, d. h. der Schaft 31 übernimmt mindestens teilweise die Aufgabe des Knorpelgewebes beim natürlichen Gelenk. Wenn man die Kalotte 29 mit einem Material beschichten könnte, das eine ausreichende Dämpfungswirkung hat, könnte man möglicherweise auf den Schaft 31 verzichten. Zweitens soll die Spongiosa durch die Einleitung von Kräften zum Wachstum animiert werden. Eine Dehnung von 0,2%, insbesondere im Bereich von 0,05% bis 0,3%, ist gut zur Förderung dieses Effekts geeignet (Siehe: Kaspar, Seidl, Ignatius, Neidlinger-Wilke, Claes, in. Der Orthopäde, 2000, 29, S. 85-90 und Frots, HM (1992): perspectives: bone's mechanical usage windows. Bone Miner 19, S. 257-271). Außerdem soll der Schaft 31 eine der Funktion der natürlichen Spongiosa möglichst weitgehend entsprechende Dämpfung haben. Der Elastizitätsmodul des Schaftes 31 muß größer sein als der Elastizitätsmodul der Spongiosa im Bereich der Resektionsflächen des Femurkopfes 7.

Aus Fig. 3a (als ein mögliches Ausführungsbeispiel) ist auch ersichtlich, dass der Schaft 31 an einer Kortikalis 23 in Bereich des Oberschenkelhalses 5 anliegt oder zumindest bis in ihre unmittelbare Nähe reicht. Dadurch werden die im Schaft 31 verbleibenden Kräfte in die Kortikalis 23 abgeführt. Aus Figur 3a ist weiter ersichtlich, dass die Längsachse des Schaftes 31 nicht parallel oder koaxial zur Längsachse 11 des Oberschenkelhalses 5 verläuft. Die Längsachse des Schaftes 31 verläuft vielmehr in der Richtung der Hauptbeanspruchungen. Diese Richtung kann, bspw. bei einem Querschnitt durch ein Präparat durch die Ausrichtung der Trabekel (nicht dargestellt) sichtbar gemacht werden. Durch diese Ausrichtung des Schaftes 31 wird auch nach der Implantation der Hüftgelenksendoprothese 27 in den Oberschenkelhals 5 die physiologische Belastung geringstmöglich gestört.

Die mit der Kalotte 29 zusammenwirkende Gelenkpfanne ist in Figur 3 nicht dargestellt. Die Verankerung dieser Gelenkpfanne im Hüftknochen kann gemäß dem Stand der Technik erfolgen.

In Fig. 3b ist der Schaft 31 teilweise aufgeschnitten dargestellt. Dadurch wird die Verbindung zwischen Schaft 31 und Kalotte 29 sichtbar. Es hat sich nämlich aus verschiedenen Gründen als vorteilhaft erwiesen, wenn Kalotte 29 und Schaft 31 zweiteilig ausgeführt werden. Dabei sind die optimale Materialwahl, der Fertigung und der Implantation wichtige Gründe, die für die zweiteilige Ausführung sprechen.

An einem Ende des Schaftes 31 ist eine konische Ausnehmung 39 vorgesehen. Kegelwinkel und Durchmesser (beide nicht eingetragen in Fig. 3b) der Ausnehmung 39 sind so bemessen, dass sie die von der Kalotte 29 in den ebenfalls nicht dargestellten Oberschenkelknochen eingeleiteten Kräfte sicher übertragen können. Die zugehörige Kalotte 29 ist in Fig 3b ebenfalls geschnitten dargestellt. Die Kalotte 29 weist einen Zapfen 41 auf, der zusammen mit der Ausnehmung 39 eine Klemmverbindung zwischen Kalotte 29 und Schaft 31 ermöglicht. Fig. 3b zeigt Kalotte 29 und Schaft 31 in zusammengebautem Zustand. Der modulare Aufbau des zweiten Ausführungsbeispiels ermöglicht die getrennte Herstellung und Optimierung von Schaft 31 und Kalotte 29 hinsichtlich Material, Herstellungsverfahren, Oberflächenbehandlung und - beschichtung. Der Übergang zwischen Schaft 31 und Innenfläche 35 kann zur Verminderung der Kerbwirkung gerundet ausgeführt werden, wie dies in Fig. 3b der Fall ist.

Es ist auch möglich, den Schaft 31 fachwerkartig auszugestalten, wobei dieses Fachwerk aus Stäben und Knoten besteht, die in verschiedene Raumrichtungen orientiert sind (nicht dargestellt). Dabei empfiehlt es sich, wie bei jedem Fachwerk, in Richtung der auftretenden Kräfte Stäbe vorzusehen. Dann wird die Festigkeit des Fachwerks bei niedrigstem Gewicht optimal.

Als vorteilhaft haben sich folgende Abmessungen erwiesen:

| **Ort** | **Durchmesser der Streben** | **Durchmesser der Öffnungen** |
|---|---|---|
| proximales Drittel | ca. 0,15 mmm | ca. 1mm |
| mittleres Drittel | ca. 0,2 mm | ca. 2 mm |
| distales Drittel | ca. 0,25 mm | ca. 3 mm |

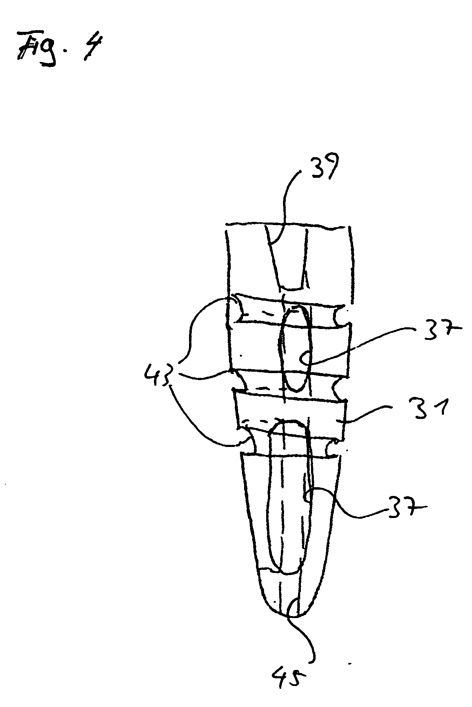

Fig. 4 zeigt ein zweites Ausführungsbeispiel eines Schafts 31. Dieses Ausführungsbeispiel weist zusätzlich zu den Öffnungen 37 noch umlaufende Nuten 43 und eine Längsbohrung 45 auf. In die Ausnehmung 39 kann eine Kalotte 29 (nicht dargestellt) gemäß Fig. 3 eingesetzt werden.

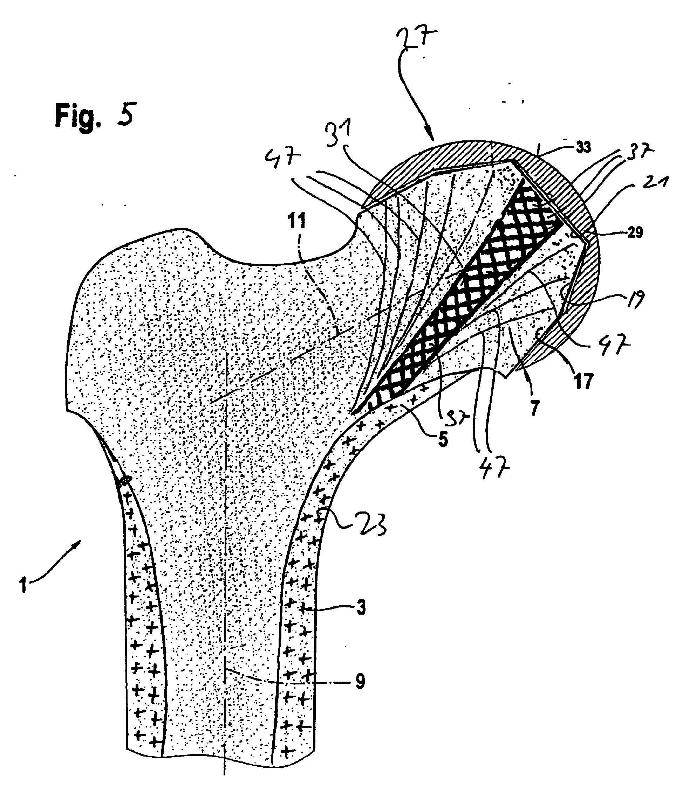

Figur 5 zeigt ein weiteres Ausführungsbeispiel einer erfindungsgemäßen Hüftgelenksendoprothese 27 im Schnitt. Dabei ist die Kalotte 29 nahezu identisch ausgeführt wie die anhand der Fign. 3a und 3b beschriebene Kalotte 29. Der wesentliche Unterschied zu dem ersten Ausführungsbeispiel gemäß Fig. 3 besteht im Schaft 31. Der Schaft 31 ist so gestaltet, dass er die Krafteinleitung in die Spongiosa des Kopfes 7 weiter verbessert. Dazu muss man vorausschicken, dass die Spongiosa aus Trabekeln (nicht dargestellt) besteht, die entlang der Hauptspannungstrajektorien 47 ausgerichtet sind. Bei einem gesunden Hüftgelenk wird von den Trabekeln ein Impuls aufgenommen und ein Teil davon über die benachbarte Trabekelstruktur (nicht dargestellt) in die Kortikalis 23 eingeleitet. Dadurch wird die mechanische Beanspruchung in der Spongiosa des Oberschenkelhalses 5 reduziert. Der erfindungsgemäße Schaft 31 gemäß Fig. 5 ahmt nun die Struktur der Spongiosa im Bereich der Hauptspannungstrajektorien 47 nach. Proximal ist er trichterförmig, verjüngt sich jedoch distal und kontaktiert schließlich die mediale Kortikalis 23. Er ist gitterförmig, z. B. wie ein 2 oder 3-dimensionales Gewebe, aufgebaut mit Öffnungen 37, deren Durchmesser etwa der Porengröße der Spongiosa entspricht. Öffnungen 37 mit einem Durchmesser zwischen 1 mm und 4 mm haben sich als vorteilhaft erwiesen.

Der Schaft 41 ist rotationssymmetrisch aufgebaut und schließt bündig mit der Stirnfläche 21 des Kopfes 7 ab. An seinem anderen Ende ist er schräg angeschnitten und liegt idealerweise auf der Kortikalis 23 auf.

Zwischen der Kalotte 29 und dem Schaft 31 gibt es keine Verbindung über eine Ausnehmung 39 und einen Zapfen 41. Die Kraft wird vielmehr von der Kalotte 29 dadurch auf den Schaft 31 übertragen, dass die der Stirnfläche 21 entsprechende Fläche der Kalotte 29 spielfrei auf dem Schaft 31 aufliegt und somit Druckkräfte von der Kalotte 29 auf den Schaft 31 übertragen werden können. Die Konstruktion der Kalotte ist mit dem Schaft 31 so abgestimmt, dass das System statisch neutral, aber dynamisch aktiv ist.

Auch bei diesem Ausführungsbeispiel verläuft die Längsachse des Schafts 31 in etwa in Richtung der Haupttrajektorien 47, d. h. der Hauptangriffsrichtung der auf den Oberschenkelhals 5 wirkenden Kräfte.

Durch die filigrane maschenartige Struktur des Schaftes 31 ist dieser besonders weich und nachgiebig, so dass eine sehr sanfte Krafteinleitung vom Schaft 31 in die ihn umgebende Spongiosa erfolgt. Außerdem kann die Spongiosa wegen der sehr großen Zahl von Öffnungen 37 im Schaft 31 sehr gut in den Schaft 31 einwachsen und sich innig mit ihm verbinden.

Der Schaft 31 ist dennoch etwas steifer als die ihn umgebende Spongiosa und zwar gerade soviel, dass die Stöße, welche von der Kalotte 29 zu einem so großen Teil vom Schaft 31 aufgenommen und an die Kortikalis 23 weitergeleitet werden, dass eine Schädigung der Spongiosa im Bereich des Oberschenkelhalses nicht erfolgt.

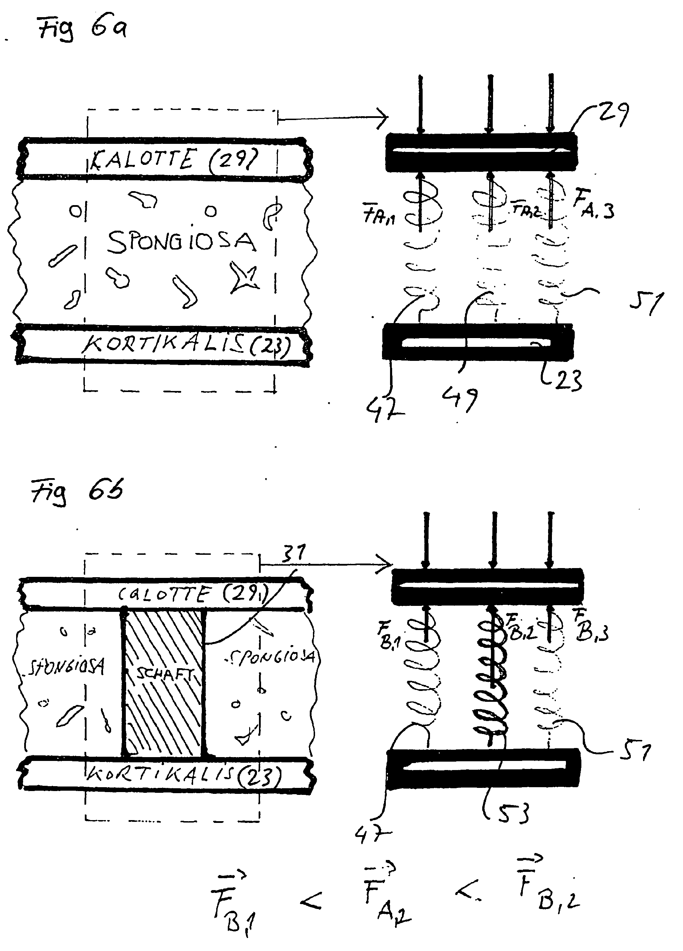

Anhand der Fign. 6a und 6b soll das Wirkungsprinzip der erfindungsgemäßen Hüftgelenksendoprothese modellhaft dargestellt werden. Dabei besteht das Modell aus drei Elementen, der Kalotte 29, der Kortikalis 23, des Oberschenkelhalses 5 und der dazwischen angeordneten Spongiosa. Wenn man nun vereinfacht die Kalotte 29 und die Kortikalis 23 jeweils als starre Körper ansieht, dann wird die gesamte Elastizität und Dämpfung von der Spongiosa übernommen. In der Fig. 6a ist die elastische Eigenschaft der Spongiosa durch drei Federn 47, 49 und 51 mit gleicher Federrate veranschaulicht. Die über die Kalotte 29 eingeleiteten Kräfte rufen in den Federn 47, 49 und 51 entsprechend gleich große Gegenkräfte F_{A,1} = F_{A,2} = F_{A,3} hervor.

In der Fig. 6b ist das Modell geringfügig variiert, indem nämlich zwischen Kalotte 29 und Kortikalis 23 zusätzlich zu der Spongiosa ein Schaft 31 vorgesehen ist, dessen Steifigkeit etwas höher ist als die der ihn umgebenden Spongiosa. Übertragen auf das Modell mit drei Federn, welches auf der rechten Seite von Fig. 6b dargestellt ist, bedeutet dies, dass die mittlere Feder 53 eine größere Federrate aufweist als die Federn 47 und 51, welche die den Schaft 31 umgebende Spongiosa repräsentieren. Dies bedeutet, dass die von den Federn 47, 51 und 53 übertragenen Kräfte nicht gleich groß sind. Es ist vielmehr so, dass die von der Feder 47 übertragene Kraft F_{B,1} und die von der Feder 51 übertragene Kraft F_{B,3} kleiner als die von der Feder 53 übertragene Kraft F_{B,2} sind. Da unter der Voraussetzung, dass eine gleich große äußere Kraft auf die Kalotte 29 einwirkt, die Summe F_{B,1} + F_{B,2} + F_{B,3} jedoch gleich groß ist wie die Kräfte F_{A,1} + F_{A,2} + F_{A,3} (siehe Fig. 6a) bedeutet dies nichts anderes, als dass der Schaft 31 zur Entlastung der ihn umgebenden Spongiosa führt, da die Kräfte F_{B,1} und F_{B,3} durch das Vorhandensein des Schafts 31 mit einer höheren Steifigkeit als die ihn umgebende Spongiosa verringert werden.

Durch die gitterartige Struktur des Schaftes 31 kann auf die Verwendung von Zement verzichtet werden, was große Vorteile hinsichtlich Festigkeit und Beständigkeit der Verankerung der Hüftgelenksendoprothese im Femur mit sich bringen. Die Vorteile zementfreier Verankerungen sind hinlänglich bekannt, allerdings ist es noch nicht gelungen, einen Oberflächenersatz für das proximale Femur zementfrei dauerhaft im Hals 5 zu befestigen.

## Patentansprüche

1. Hüftgelenksendoprothese als Oberflächenersatz des proximalen Femurs mit einer Kalotte (29), wobei die Kalotte (29) eine mit einer Gelenkpfanne zusammenwirkende Aussenfläche (33) und eine.Innenfläche (35) aufweist und mit einem Schaft (31), wobei der Schaft (31) mit der Kalotte (29) verbindbar ist und dass der Schaft (31) eine gitterartige Struktur aufweist, **dadurch gekennzeichnet, dass** die Längsachse des Schaftes (31) in Richtung der höchsten Beanspruchung des Oberschenkelhalses (5) verläuft.

2. Hüftgelenksendoprothese nach Anspruch 1, **dadurch gekennzeichnet, dass** die Steifigkeit des Schaftes (31), insbesondere in Längsrichtung des Schaftes (31), größer als die den Schaft (31) umgebende Spongiosa ist.

3. Hüftgelenksendoprothese nach Anspruch 1 oder 2, **dadurch gekennzeichnet, dass** die Steifigkeit des Schaftes (31), insbesondere in Längsrichtung des Schaftes (31), kleiner als die Kortikalis des Oberschenkelhalses ist.

4. Hüftgelenksendoprothese nach einem der vorhergehenden Ansprüche, **dadurch gekennzeichnet, dass** sich die gitterartige Struktur des Schaftes (31) aus Öffnungen (37) im Schaft (31), Maschen oder Stäben und Streben zusammensetzt.

5. Hüftgelenksendoprothese nach einem der vorhergehenden Ansprüche, **dadurch gekennzeichnet, das**s die gitterartige Struktur Verdickungen und/oder Vertiefungen aufweisen.

6. Hüftgelenksendoprothese nach einem der vorhergehenden Ansprüche, **dadurch gekennzeichnet, dass** das erste Drittel des Schaftes (31) biegesteif mit der Kalotte verbunden ist, und dass das erste Drittel des Schaftes (31) in Längsrichtung eine in den Grenzen der Ansprüche 2 und 3 liegende Steifigkeit aufweist.

7. Hüftgelenksendoprothese nach einem der vorhergehenden Ansprüche, **dadurch gekennzeichnet, dass** das zweite Drittel des Schaftes (31) biegeweich ist, und dass das zweite Drittel des Schaftes (31) in Längsrichtung eine in den Grenzen der Ansprüche 2 und 3 liegende Steifigkeit aufweist.

8. Hüftgelenksendoprothese nach einem der vorhergehenden Ansprüche, **dadurch gekennzeichnet, dass** das dritte Drittel des Schaftes (31) biegeweich ist, und dass das dritte Drittel des Schaftes (31) in Längsrichtung über der Steifigkeit der Kortikalis (23) des Oberschenkelhalses (5) liegende Steifigkeit aufweist.

9. Hüftgelenksendoprothese nach einem der vorhergehenden Ansprüche, **dadurch gekennzeichnet, dass** die Stäbe des Schaftes (31) im proximalen Drittel einen Durchmesser von ca. 0,15 mm und einen Abstand zueinander von ca. 1 mm, im mitteleren Drittel einen Durchmesser von ca, 0,2 mm und einen Abstand zueinander von ca. 2 mm und im distalen Drittel einen Durchmesser von ca. 0,25 mm und einen Abstand zueinander von ca. 3 mm haben.

10. Hüftgelenksendoprothese nach einem der vorhergehenden Ansprüche, **dadurch gekennzeichnet, dass** der Schaft (31) in implantiertem Zustand bei den auftretenden Belastungen an der Oberfläche eine Dehnung von 0,2%, insbesondere von 0,05% bis 0,3% hat.

11. Hüftgelenksendoprothese nach einem der vorhergehenden Ansprüche, **dadurch gekennzeichnet, dass** die Oberfläche des Schaftes (31) porös oder periplasmal ist.

12. Hüftgelenksendoprothese nach einem der vorhergehenden Ansprüche, **dadurch gekennzeichnet, dass** die Oberfläche des Schaftes (31) hydroxyapatit ist.

13. Hüftgelenksendoprothese nach einem der vorhergehenden Ansprüche, **dadurch gekennzeichnet, dass** die Innenfläche (35) der Oberfläche eines Kegelstumpfs (17, 19, 21), insbesondere eines doppelten Kegelstumpfs, entspricht.

14. Hüftgelenksendoprothese nach einem der vorhergehenden Ansprüche, **dadurch gekennzeichnet, dass** der oder die Durchmesser der Innenfläche (35) so bemessen sind, dass sie kleiner sind als die Kortikalis (15) des Kopfes (7) des Femurs (1).

15. Hüftgelenksendoprothese nach einem der vorhergehenden Ansprüche, **dadurch gekennzeichnet, dass** die Kalotte (29) und der Schaft (31) durch eine Klemmverbindung miteinander verbunden sind.

16. Hüftgelenksendoprothese nach Anspruch 15, **dadurch gekennzeichnet, dass** der Schaft (31) eine konische Ausnehmung (39) aufweist, und dass die Kalotte (29) einen Zapfen (41) aufweist, und dass die Ausnehmung (39) und der Zapfen (41) durch Klemmung verbindbar sind.

17. Hüftgelenksendoprothese nach Anspruch 16, **dadurch gekennzeichnet, dass** der Querschnitt der konischen Ausnehmung (39) und des Zapfens (41) rund, polygonal, rechteckig oder quadratisch ist.

18. Hüftgelenksendoprothese nach einem der vorhergehenden Ansprüche, **dadurch gekennzeichnet, dass** die Kalotte (29) und der Schaft (31) einteilig ausgeführt sind.

19. Hüftgelenksendoprothese nach einem der vorhergehenden Ansprüche, **dadurch gekennzeichnet, dass** die Kalotte (29) und der Schaft (31) zweiteilig ausgeführt sind, und dass die Kalotte (29) in implantiertem Zustand auf dem Schaft (31) aufliegt.

20. Hüftgelenksendoprothese nach einem der vorhergehenden Ansprüche, **dadurch gekennzeichnet, dass** die Aussenfläche (33) der Kalotte (29) mit einer dämpfenden, verschleißmindernden und reibungsverringernden Beschichtung versehen ist.

21. Hüftgelenksendoprothese nach einem der vorhergehenden Ansprüche, **dadurch gekennzeichnet, dass** die Hüftgelenksendoprothese mindestens teilweise aus Titan, insbesondere TiAl6V4, einer Stahl-Legierung oder CoCr, durch Gießen und/oder Schmieden, aus Sintermetall oder einer porösen Keramik oder biologisch abbaubarem Material bestehen.

22. Hüftgelenksendoprothese nach einem der vorhergehenden Ansprüche, **dadurch gekennzeichnet, dass** die Konstruktion der Kalotte mit dem Schaft so abgestimmt ist, dass das System statisch neutral, aber dynamisch aktiv ist.

## Claims

1. Hip-joint endoprosthesis forming a replacement for the surface of the proximal femur, having a part-sphere (29), the part-sphere (29) having an outer surface (33) which co-operates with a joint socket and an inner surface (35), and having a shank (31), the shank (31) being able to be connected to the part-sphere (29) and that the shank (31) has a lattice-like structure, **characterised in that** the longitudinal axis of the shank (31) extends in the direction of the highest stress on the neck (5) of the femur.

2. Hip-joint endoprosthesis according to claim 1, **characterised in that** the stiffness of the shank (31), particularly in the longitudinal direction thereof, is greater than that of the spongiosa surrounding the shank (31)

3. Hip-joint endoprosthesis according to claim 1 or 2, **characterised in that** the stiffness of the shank (31), particularly in the longitudinal direction thereof, is smaller than that of the cortical bone of the neck of the femur.

4. Hip-joint endoprosthesis according to one of the foregoing claims, **characterised in that** the lattice-like structure of the shank (31) is composed of openings (37) in the shank (31), meshes or rods and struts.

5. Hip-joint endoprosthesis according to one of the foregoing claims, **characterised in that** the lattice-like structure has thickenings and/or depressions.

6. Hip-joint endoprosthesis according to one of the foregoing claims, **characterised in that** the first third of the shank (31) is connected to the part-sphere in such a way as to be stiff in bending, and **in that** the first third of the shank (31) is of a stiffness in the longitudinal direction which is within the limits given in claims 2 and 3.

7. Hip-joint endoprosthesis according to one of the foregoing claims, **characterised in that** the second third of the shank (31) is soft in bending, and **in that** the second third of the shank (31) is of a stiffness in the longitudinal direction which is within the limits given in claims 2 and 3.

8. Hip-joint endoprosthesis according to one of the foregoing claims, **characterised in that** the third third of the shank (31) is soft in bending, and **in that** the third third of the shank (31) is of a stiffness in the longitudinal direction which is above the stiffness of the cortical bone (23) of the neck (5) of the femur.

9. Hip-joint endoprosthesis according to one of the foregoing claims, **characterised in that**, in the proximal third, the diameter of the rods of the shank (31) is approximately 0.15 mm and the distance between them approx. 1 mm, in the centre third the diameter of the rods is approximately 0.2 mm and the distance between them approximately 2 mm and in the distal third the diameter of the rods is approximately 0.25 mm and the distance between them approximately 3 mm.

10. Hip-joint endoprosthesis according to one of the foregoing claims, **characterised in that**, in the implanted state and under the loads which occur, the shank (31) has, at the surface, an expansion of 0.2% and in particular of 0.05% to 0.3%.

11. Hip-joint endoprosthesis according to one of the foregoing claims, **characterised in that** the surface of the shank (31) is porous or periplasmal.

12. Hip-joint endoprosthesis according to one of the foregoing claims, **characterised in that** the surface of the shank (31) is hydroxyapatite.

13. Hip-joint endoprosthesis according to one of the foregoing claims, **characterised in that** the inner surface (35) corresponds to the surface of a truncated cone (17, 19, 21), and in particular of a double truncated cone,

14. Hip-joint endoprosthesis according to one of the foregoing claims, **characterised in that** the diameter or diameters of the inner surface (35) are of a size such that they are smaller than the cortical bone (15) of the head (7) of the femur (1).

15. Hip-joint endoprosthesis according to one of the foregoing claims, **characterised in that** the part-sphere (29) and the shank (31) are connected together by an interference connection.

16. Hip-joint endoprosthesis according to claim 15, **characterised in that** the shank (31) has a conical recess (39) and **in that** the part-sphere (29) has a spigot (41) and **in that** the recess (39) and the spigot (41) are connectable by interference

17. Hip-joint endoprosthesis according to claim 16, **characterised in that** the cross-section of the conical recess (39) and the spigot (41) is circular, polygonal, rectangular or square.

18. Hip-joint endoprosthesis according to one of the foregoing claims, **characterised in that** the part-sphere (29) and the shank (31) are produced in one piece.

19. Hip-joint endoprosthesis according to one of the foregoing claims, **characterised in that** the part-sphere (29) and the shank (31) are produced in two pieces and **in that** the part-sphere (29) rests on the shank (31) in the implanted state.

20. Hip-joint endoprosthesis according to one of the foregoing claims, **characterised in that** the outer surface (33) of the part-sphere (29) is provided with a coating which is damping, reduces wear and reduces friction.

21. Hip-joint endoprosthesis according to one of the foregoing claims, **characterised in that** at least part of the hip-joint endoprosthesis is composed of titanium, and in particular TiAl6V4, a steel alloy or CoCr, as a result of casting and/or forging, of sintered metal or a porous ceramic material or biodegradable material.

22. Hip-joint endoprosthesis according to one of the foregoing claims, **characterised in that** the design of the part-sphere is matched to the shank in such a way that the system is statically neutral but dynamically active.

## Revendications

1. Endoprothèse pour l'articulation de la hanche en remplacement superficiel du fémur proximal avec une calotte (29), la calotte (29) comportant une face extérieure (33) qui coopère avec un cotyle et une face intérieure (35) et avec une tige (31), la tige (31) pouvant être reliée à la calotte (29), et la tige (31) présentant une structure semblable à un grillage, **caractérisée en ce que** l'axe longitudinal de la tige (31) s'étend dans le sens de la sollicitation maximale du col du fémur (5).

2. Endoprothèse pour l'articulation de la hanche selon la revendication 1, **caractérisée en ce que** la rigidité de la tige (31), en particulier dans le sens longitudinal de la tige (31), est supérieure au tissu spongieux entourant la tige (31).

3. Endoprothèse pour l'articulation de la hanche selon la revendication 1 ou 2, **caractérisée en ce que** la rigidité de la tige (31), en particulier dans le sens longitudinal de la tige (31), est inférieure à la corticale du col du fémur.

4. Endoprothèse pour l'articulation de la hanche selon l'une quelconque des revendications précédentes, **caractérisée en ce que** la structure semblable à un grillage de la tige (31) est constituée d'ouvertures (37) dans la tige (31), de mailles ou de barres et de renforts.

5. Endoprothèse pour l'articulation de la hanche selon l'une quelconque des revendications précédentes, **caractérisée en ce que** la structure semblable à un grillage de la tige (31) comporte des épaississements et/ou des creux.

6. Endoprothèse pour l'articulation de la hanche selon l'une quelconque des revendications précédentes, **caractérisée en ce que** le premier tiers de la tige (31) est relié à la calotte de manière rigide à la flexion et **en ce que** le premier tiers de la tige (31) dans le sens longitudinal présente une rigidité comprise dans les limites des revendications 2 et 3.

7. Endoprothèse pour l'articulation de la hanche selon l'une quelconque des revendications précédentes, **caractérisée en ce que** le deuxième tiers de la tige (31) est non rigide et **en ce que** le deuxième tiers de la tige (31) dans le sens longitudinal présente une rigidité comprise dans les limites des revendications 2 et 3.

8. Endoprothèse pour l'articulation de la hanche selon l'une quelconque des revendications précédentes, **caractérisée en ce que** le troisième tiers de la tige (31) est non rigide et **en ce que** le troisième tiers de la tige (31) dans le sens longitudinal présente une rigidité supérieure à la rigidité de la corticale (23) du col du fémur (5).

9. Endoprothèse pour l'articulation de la hanche selon l'une quelconque des revendications précédentes, **caractérisée en ce que** les barres de la tige (31) présentent un diamètre d'environ 0,15 mm et une distance d'environ 1 mm les unes par rapport aux autres dans le tiers proximal, un diamètre d'environ 0,2 mm et une distance d'environ 2 mm les unes par rapport aux autres dans le tiers moyen et un diamètre d'environ 0,25 mm et une distance d'environ 3 mm les unes par rapport aux autres dans le tiers distal.

10. Endoprothèse pour l'articulation de la hanche selon l'une quelconque des revendications précédentes, **caractérisée en ce que** la tige (31) à l'état implanté présente une dilatation de 0,2%, en particulier comprise entre 0,05% et 0,3%, dans le cas des charges s'exerçant sur la surface.

11. Endoprothèse pour l'articulation de la hanche selon l'une quelconque des revendications précédentes, **caractérisée en ce que** la surface de la tige (31) est poreuse ou périplasmique.

12. Endoprothèse pour l'articulation de la hanche selon l'une quelconque des revendications précédentes, **caractérisée en ce que** la surface de la tige (31) est de l'hydroxyapatite.

13. Endoprothèse pour l'articulation de la hanche selon l'une quelconque des revendications précédentes, **caractérisée en ce que** la face intérieure (35) correspond à la surface d'un cône tronqué (17, 19, 21), en particulier d'un cône tronqué double.

14. Endoprothèse pour l'articulation de la hanche selon l'une quelconque des revendications précédentes, **caractérisée en ce que** le diamètre ou les diamètres de la face intérieure (35) est dimensionné ou sont dimensionnés de telle manière qu'ils sont plus petits que la corticale (15) de la tête (7) du fémur (1).

15. Endoprothèse pour l'articulation de la hanche selon l'une quelconque des revendications précédentes, **caractérisée en ce que** la calotte (29) et la tige (31) sont reliées l'une à l'autre par un collier de serrage.

16. Endoprothèse pour l'articulation de la hanche selon la revendication 15, **caractérisée en ce que** la tige (31) comporte un évidement conique (39) et **en ce que** la calotte comporte un tenon (41), et **en ce que** l'évidement (39) et le tenon (41) peuvent être reliés par serrage.

17. Endoprothèse pour l'articulation de la hanche selon la revendication 16, **caractérisée en ce que** la section transversale de l'évidement conique (39) et du tenon (41) est ronde, polygonale, rectangulaire ou carrée.

18. Endoprothèse pour l'articulation de la hanche selon l'une quelconque des revendications précédentes, **caractérisée en ce que** la calotte (29) et la tige (31) sont réalisées en une seule partie.

19. Endoprothèse pour l'articulation de la hanche selon l'une quelconque des revendications précédentes, **caractérisée en ce que** la calotte (29) et la tige (31) sont réalisées en deux parties et **en ce que** la calotte (29) à l'état implanté s'appuie sur la tige (31).

20. Endoprothèse pour l'articulation de la hanche selon l'une quelconque des revendications précédentes, **caractérisée en ce que** la face extérieure (33) de la calotte (29) est munie d'un revêtement servant à l'amortissement, à la réduction de l'usure et à la diminution de la friction.

21. Endoprothèse pour l'articulation de la hanche selon l'une quelconque des revendications précédentes, **caractérisée en ce que** l'endoprothèse pour l'articulation de la hanche est constituée au moins partiellement de titane, en particulier de TiAl6V4, d'un alliage d'acier ou de CoCr, par coulée et/ou forgeage, de métal fritté ou d'une céramique poreuse ou d'un matériau biodégradable.

22. Endoprothèse pour l'articulation de la hanche selon l'une quelconque des revendications précédentes, **caractérisée en ce que** la construction de la calotte avec la tige est adaptée de telle manière que le système est statiquement neutre, mais dynamiquement actif.
